# EUROPÄISCHE PATENTSCHRIFT

(11) **EP 2 796 108 B1**
(45) Veröffentlichungstag und Bekanntmachung des Hinweises auf die Patenterteilung: **23.08.2017**
(21) Anmeldenummer: 13165080.6
(22) Anmeldetag: 24.04.2013
(51) Int. Cl.: A61C 1/05, A61C 1/08, A61B 17/00

(54) **Medizinisches, insbesondere dentales, Handstück**
Medical, in particular dental, handpiece
Pièce à main médicale, en particulier dentaire

(43) Veröffentlichungstag der Anmeldung: 29.10.2014
(73) Patentinhaber: W & H Dentalwerk Bürmoos GmbH, 5111 Bürmoos (AT)
(72) Erfinder: Schmiedlechner, Karl, 5121 Ostermiething (AT); Hofbauer, Manfred, 5111 Bürmoos (AT)
(74) Vertreter: Benda, Ralf

(56) Entgegenhaltungen:
- US-A1- 2007 121 786
- US-A1- 2008 131 835

## Beschreibung

Die vorliegende Erfindung betrifft ein medizinisches, insbesondere dentales, Handstück, mit einer Außenhülse und einer Beleuchtungsvorrichtung, die ein optisches Halbleiterelement aufweist und die an einer Öffnung der Außenhülse angeordnet ist.

Ein derartiges medizinisches, insbesondere dentales, Handstück ist zum Beispiel aus der Patentanmeldung US 2008/0131835 A1 bekannt. Die Fixierung der Beleuchtungsvorrichtung in der Öffnung der Außenhülse wird bei diesem Handstück dadurch bewirkt, dass einen Fortsatz oder Verbindungsabschnitt des Kopfteils des Handstücks die Beleuchtungsvorrichtung in die Öffnung drückt. In dem Fortsatz des Kopfteils sind des Weiteren verschiedene Fluidleitungen vorgesehen, unter anderem eine Rückluftleitung, welche die Antriebsluft von einer pneumatischen Antriebsvorrichtung des Handstücks ableitet. US 2007/121786 A1 offenbart ein Handstück nach der Präambel von Anspruch 1. Der vorliegenden Erfindung liegt die Aufgabe zugrunde, eine alternative Fixierung der Beleuchtungsvorrichtung in der Öffnung der Außenhülse zu schaffen, die es insbesondere ermöglicht, den Innendurchmesser der Rückluftleitung zu vergrößern, um eine möglichst optimale Ableitung des Antriebsgases mit geringem Rückstau zu ermöglichen.

Diese Aufgabe wird gemäß der vorliegenden Erfindung durch ein medizinisches, insbesondere dentales, Handstück, nach Anspruch 1 gelöst. Durch das Vorsehen des Stiftes zum Fixieren des optischen Halbleiterelements oder der Beleuchtungsvorrichtung entfällt die Notwendigkeit, den Fortsatz oder Verbindungsabschnitt des Kopfteils derart zu bemessen, dass er das optische Halbleiterelement oder die Beleuchtungsvorrichtung fixiert. Besonders bevorzugt ist es dadurch möglich, das optische Halbleiterelement oder die Beleuchtungsvorrichtung in dem Kopfteil, insbesondere in dem Verbindungsabschnitt, anzuordnen. Dies ermöglicht in beiden Fällen in vorteilhafter Weise zumindest einen Innendurchmesser einer Fluidleitung des Handstücks, insbesondere den Innendurchmesser der Rückluftleitung, zu vergrößern.

Vorzugsweise umfasst die Aufnahme für den Stift eine Querbohrung und / oder eine Ausnehmung, deren Längsachse(n) insbesondere gewinkelt zur Mittelachse der Außenhülse angeordnet ist / sind. Vorzugsweise ist zumindest ein Teil der Aufnahme für den Stift, insbesondere der Querbohrung oder der Ausnehmung, innerhalb der Außenhülse oder im Inneren des Handstücks vorgesehen. Vorzugsweise ist zumindest ein Teil der Aufnahme, insbesondere der Querbohrung oder der Ausnehmung, in einem Bauteil des Handstücks vorgesehen, insbesondere in der Außenhülse und / oder in einem mit der Außenhülse (un)lösbar verbundenen Bauteil. Vorzugsweise umfasst die Aufnahme, insbesondere die Querbohrung oder die Ausnehmung, mehrere Abschnitte, die insbesondere voneinander getrennt sind.

Das Handstück ist vorzugsweise als pneumatisch antreibbares Handstück ausgebildet und umfasst insbesondere eine durch ein Druckgas antreibbare Antriebsvorrichtung, zum Beispiel ein Laufrad. Die Antriebsvorrichtung ist insbesondere mit einer lösbaren Werkzeughaltevorrichtung zum Halten des mit dem Handstück verbindbaren Werkzeugs operativ verbunden. Das Handstück ist insbesondere über eine endseitige Kupplungsvorrichtung mit einer Druckgasquelle verbindbar. Das Handstück weist bevorzugt einen Kopfteil mit der Werkzeughaltevorrichtung zum Halten des mit dem Handstück verbindbaren Werkzeugs und einen an den Kopfteil anschließenden (mit einer Hand haltbaren) Griffteil auf, wobei die Beleuchtungsvorrichtung und der Stift im Wesentlichen in dem Kopfteil vorgesehen sind. Damit ist es in vorteilhafter Weise möglich, die Beleuchtungsvorrichtung sehr nahe an dem Werkzeug und / oder der Behandlungsstelle vorzusehen. Besonders bevorzugt sind die Beleuchtungsvorrichtung und / oder der Stift zumindest teilweise in einem Verbindungsabschnitt des Kopfteils, der zumindest teilweise in den Griffteil einfügbar ist, vorgesehen.

Bevorzugt ist der Kopfteil von dem Griffteil lösbar, wobei durch das Verbinden des Kopfteils mit dem Griffteil der Stift zwischen der Außenhülse und der Beleuchtungsvorrichtung klemmbar ist. Damit ist in vorteilhafter Weise kein weiteres Bauteil zum Fixieren der Beleuchtungsvorrichtung notwendig. Des Weiteren ist damit eine einfache Montage des Stifts und eine einfache Fixierung der Beleuchtungsvorrichtung gewährleistet. Dazu weist besonders bevorzugt das Kopfteil einen Verbindungsabschnitt auf, der zumindest teilweise in den Griffteil einfügbar ist, wobei in einer (äußeren) Umfangswand des Verbindungsabschnitts, vorzugsweise in einem in den Griffteil einfügbaren Bereich des Verbindungsabschnitts, zumindest ein Abschnitt der Aufnahme für den Stift, insbesondere der Querbohrung oder der Ausnehmung, vorgesehen ist. Durch das Einfügen des Verbindungsabschnitts in den Griffteil wird damit bewirkt, dass die Außenhülse, insbesondere deren Innenseite, den Stift in Richtung oder auf die Beleuchtungsvorrichtung drückt. Dem entsprechend ist die Außenhülse, insbesondere deren Innenseite, ausgebildet, den Stift in Richtung oder auf die Beleuchtungsvorrichtung zu drücken und / oder es sind die Außenhülse, insbesondere deren Innenseite, und der Stift ausgebildet, die Beleuchtungsvorrichtung in der Öffnung der Außenhülse zu fixieren. Alternativ ist es selbstverständlich möglich, den Stift durch ein Federelement in Richtung der Beleuchtungsvorrichtung vorzuspannen.

Die Außenhülse des Handstücks ist vorzugsweise länglich und hohl oder rohrförmig ausgebildet, so dass sie insbesondere einen Innenraum bildet. Die längliche Außenhülse erstreckt sich vorzugsweise entlang ihrer Mittelachse. Die Außenhülse ist vorzugsweise derart gebogen, dass sie zumindest zwei gewinkelt zueinander angeordnete Abschnitte aufweist. In der Außenhülse oder in deren Innenraum sind insbesondere eine oder mehrere Fluidleitungen und / oder ein elektrischer Leiter zur Versorgung der Beleuchtungsvorrichtung mit elektrischer Energie vorgesehen. Besonders bevorzugt bildet zumindest ein Abschnitt des Kopfteils, insbesondere des Verbindungsabschnitt des Kopfteils, einen Teil der Außenhülse oder einen Teil einer äußeren Oberfläche des Handstücks, an dem die Öffnung für die Beleuchtungsvorrichtung vorgesehen ist. Die Beleuchtungsvorrichtung und / oder das optische Halbleiterelement sind vorzugsweise in oder an der Öffnung der Außenhülse vorgesehen.

Bevorzugt ist der Stift ein separates und / oder von der Beleuchtungsvorrichtung oder dem optischen Halbleiterelement unabhängiges Bauteil und / oder ein von der Beleuchtungsvorrichtung lösbares Bauteil. Bevorzugt ist der Stift zwischen der Außenhülse und der Beleuchtungsvorrichtung geklemmt. Bevorzugt weist der Stift Kunststoff auf oder ist aus Kunststoff gefertigt. Bevorzugt ist der Stift gerade ausgebildet. Bevorzugt bildet der Stift ein elektrisches Isolationsmittel, insbesondere in Bezug auf eine metallische Außenhülse. Vorzugsweise ist der Winkel, den der Stift und / oder die Aufnahme des Stifts, insbesondere die Querbohrung oder die Ausnehmung, mit der Mittelachse der Außenhülse einschließt / einschließen, größer 30°, insbesondere größer 45°, besonders bevorzugt beträgt der Winkel etwa 60°-90°.

Vorzugsweise ist der Stift zumindest teilweise in einem durch die Außenhülse gebildeten Innenraum des Handstücks angeordnet. Vorzugsweise kontaktiert der Stift die Außenhülse, insbesondere deren Innenseite. Vorzugsweise durchquert der Stift im Wesentlichen den gesamten durch die Außenhülse gebildeten Innenraum, insbesondere unter Einschluss eines im Vorstehenden genannten Winkels mit der Mittelachse der Außenhülse. Alle diese Merkmale gewährleisten eine zuverlässige Befestigung der Beleuchtungsvorrichtung in der Öffnung der Außenhülse durch Klemmen des Stifts zwischen der Außenhülse und der Beleuchtungsvorrichtung oder durch Drücken des Stifts durch die Außenhülse auf die Beleuchtungsvorrichtung.

Vorzugsweise ist der Stift zumindest teilweise in einer Fluidleitung des Handstücks aufgenommen, insbesondere in einer ein Antriebsgas von einer pneumatischen Antriebsvorrichtung des Handstücks ableitenden Fluidleitung oder Rückluftleitung. Die Anordnung des Stifts in der Rückluftleitung ermöglicht in vorteilhafter Weise, den Durchmesser oder Innenraum der Rückluftleitung besonders groß zu bemessen.

Gemäß einem Ausführungsbeispiel ist einen Fortsatz vorgesehen, in dem zumindest ein Abschnitt der Aufnahme für den Stift, insbesondere der Querbohrung oder der Ausnehmung, und / oder der Stift angeordnet ist / sind, wobei der Fortsatz in den durch die Außenhülse gebildeten Innenraum oder in einen Innenraum der den Stift aufnehmenden Fluidleitung, insbesondere der Rückluftleitung, ragt. Der Fortsatz hält oder lagert oder positioniert den Stift, wodurch insbesondere eine zuverlässige Fixierung der Beleuchtungsvorrichtung gewährleistet wird. Vorzugsweise weist der Fortsatz ein freies Ende auf, das im Wesentlichen parallel zur Mittelachse der Außenhülse verläuft. Vorzugsweise weisen die Außenhülse oder die Fluidleitung, insbesondere die Rückluftleitung, im Bereich des Fortsatzes somit einen nicht runden oder nicht ovalen oder gebogenen, insbesondere S-förmigen, Querschnitt auf. Vorzugsweise ist der Fortsatz in etwa mittig in dem Querschnitt der Außenhülse angeordnet. Vorzugsweise umhüllt oder lagert der Fortsatz nur einen Teil des Stifts, so dass zumindest ein weiterer Teil des Stifts über den Fortsatz ragt. Insbesondere umhüllt oder lagert der Fortsatz einen mittleren Abschnitt des Stifts oder einen zwischen den beiden Enden des Stifts vorgesehenen Abschnitt des Stifts. Vorzugsweise ist der Fortsatz einteilig mit der Außenhülse oder der Fluidleitung ausgebildet. Vorzugsweise bildet die Außenhülse zumindest an einem Abschnitt die Rückluftleitung, insbesondere im Bereich des Fortsatzes. Vorzugsweise ist in dem Fortsatz zumindest eine weitere Fluidleitung des Handstücks zumindest teilweise vorgesehen, zum Beispiel eine Leitung zum Fördern des Antriebsgases in Richtung des pneumatischen Antriebs des Handstücks.

Vorzugsweise ist die Beleuchtungsvorrichtung, insbesondere das optische Halbleiterelement, unlösbar oder lösbar auf einer gedruckten Leiterbahn und / oder auf einem flexiblen oder biegsamen Trägerelement vorgesehen, insbesondere auf einer flexiblen oder biegsamen Platine. Vorzugsweise verbindet die gedruckte Leiterbahn und / oder das Trägerelement die Beleuchtungsvorrichtung elektrisch mit einer elektrischen Energiequelle. Vorzugsweise ist die Energiequelle als Generator ausgebildet und insbesondere in dem Handstück oder in einer an das Handstück anschließbaren Kupplungs- oder Adaptereinheit vorgesehen. Alternativ ist die Beleuchtungsvorrichtung über elektrische Leitungen mit der elektrischen Energiequelle verbunden. Vorzugsweise ist jener Abschnitt der gedruckten Leiterbahn und / oder des Trägerelements, auf dem die Beleuchtungsvorrichtung vorgesehen ist, gewinkelt zu einem anderen Abschnitt der gedruckten Leiterbahn und / oder des Trägerelements angeordnet.

Vorzugsweise kontaktiert der Stift die gedruckte Leiterplatte, auf welcher die Beleuchtungsvorrichtung vorgesehen ist. Insbesondere sind die Beleuchtungsvorrichtung und der Stift an gegenüberliegenden Seiten der gedruckten Leiterplatte vorgesehen. Alternativ kontaktiert der Stift die Beleuchtungsvorrichtung unmittelbar, zum Beispiel ein (keramisches) Trägerelement, auf dem das optische Halbleiterelement befestigt ist.

Die Beleuchtungsvorrichtung umfasst neben dem zumindest einen optischen Halbleiterelement zumindest eines der weiteren Elemente: Ein Trägerelement, auf dem das optische Halbleiterelement befestigt ist, zum Beispiel ein keramisches Trägerelement; elektrische Kontakte zur Verbindung des optischen Halbleiterelements mit der Energiequelle, zum Beispiel zumindest einen elektrischen Leiter, der das Trägerelement durchsetzt; eine das optische Halbleiterelement umgebendes Hüllelement, zum Beispiel ein Hüllelement aus Metall oder Kunststoff, insbesondere aus verformbarem Kunststoff, besonders bevorzugt ein Hüllelement mit einer Silikonverbindung; ein, zum Beispiel in dem Hüllelement vorgesehenes, Konversionsmaterial zur Wandlung der von dem optischen Halbleiterelement abgestrahlten Wellenlänge; eine, vorzugsweise in der Öffnung der Außenhülse angeordnete, Kappe, die zum Beispiel kugelig oder kuppelförmig geformt ist und die insbesondere durch eine Kunststoffschale oder Kunststoffhartschale gebildet ist. Das Hüllelement und die Kappe sind vorzugsweise transparent für sichtbares Licht ausgebildet. Die Kappe ragt vorzugsweise durch die Öffnung der Außenhülse.

Vorzugsweise drückt der Stift das das optische Halbleiterelement umgebende Hüllelement, insbesondere ein Silikonelement, unter Verformung in die Kappe der Beleuchtungsvorrichtung. Damit wird in vorteilhafter Weise ein besonders gute Übertragung des Lichts von dem Hüllelement in die Kappe erreicht und es wird vermieden, dass Verunreinigungen zwischen die Kappe und das Hüllelement gelangen. Zusätzlich oder alternativ ist es möglich, an der Kappe und / oder an dem das optische Halbleiterelement umgebenden Hüllelement und / oder zwischen der Kappe und dem Hüllelement ein Dichtmittel oder Dichtelement vorzusehen.

Vorzugsweise ist der Stift derart angeordnet, dass er und / oder eine Längsachse des Stifts im Wesentlichen rechtwinkelig zu jener Oberfläche des Trägerelements angeordnet ist, auf der das optische Halbleiterelement befestigt ist.

Die Erfindung wird nachfolgend anhand bevorzugter Ausführungsbeispiele und Bezug nehmend auf die beigefügten Zeichnungen erläutert. Es zeigt die
Figur 1 eine Schnittdarstellung durch das Vorderende eines medizinischen, insbesondere dentalen, Handstücks mit einer Beleuchtungsvorrichtung, die durch einen Stift fixiert ist.
Figur 2 eine Schnittdarstellung durch den Griffteil des Handstücks der Figur 1 mit Blick in Richtung des Kopfteils des Handstücks.

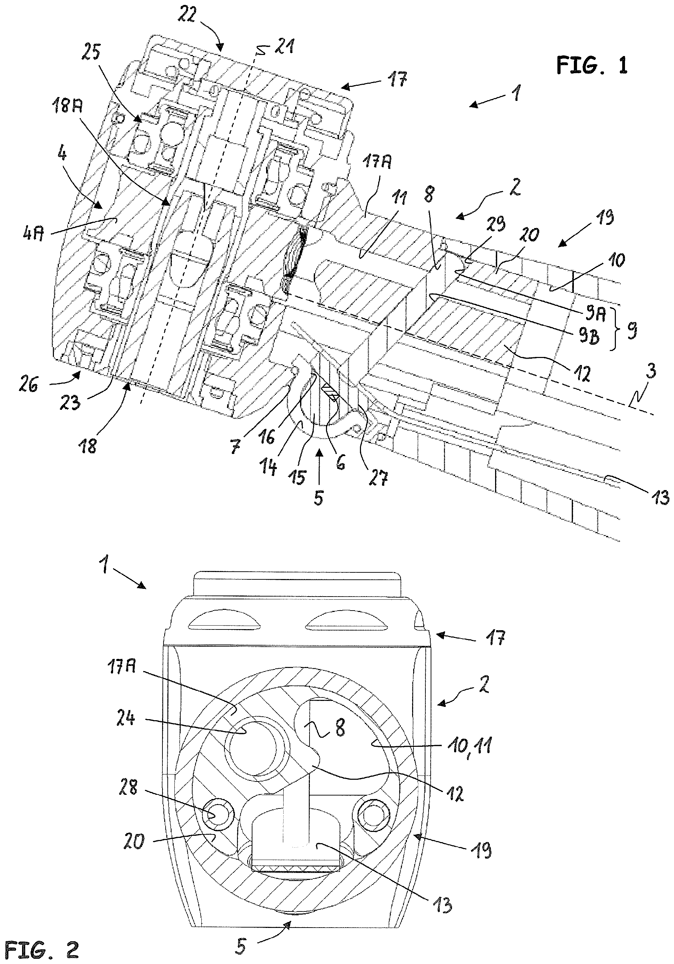

Das in den Figuren 1 und 2 dargestellte medizinische, insbesondere dentale, Handstück 1 umfasst einen Kopfteil 17 mit einer Werkzeughaltevorrichtung 18 zum Halten eines mit dem Handstück 1 verbindbaren Werkzeugs und einen an den Kopfteil 17 anschließenden Griffteil 19. Der Kopfteil 17 und der Griffteil 19 sind voneinander lösbar und insbesondere durch eine lösbare Verbindung, zum Beispiel eine Schraub- oder Steckverbindung, miteinander verbunden. Die Verbindungsstellen des Kopfteils 17 und des Griffteils 19 sind zum Beispiel als, insbesondere ringförmige(r), Anschlag, Schulter oder Stufe ausgebildet. An der Verbindungsstelle des Kopfteils 17 und des Griffteils 19 ist vorzugsweise ein Dichtelement, zum Beispiel ein O-Ring, vorgesehen. Vorzugsweise ist zur Verbindung des Kopfteils 17 und des Griffteils 19 ein, insbesondere mit dem Kopfteil 17 einteilig ausgebildetes, Verbindungsteil 17A vorgesehen, das zumindest teilweise in den Griffteil 19 einfügbar ist. Zumindest ein Teil der Außenfläche des Verbindungsteils 17A kann auch als Außenhülse 2 ausgebildet sein.

Die Werkzeughaltevorrichtung 18 umfasst eine lösbare Spannzange 18A, so dass ein Werkzeug lösbar mit dem Handstück 1 verbindbar ist. Die Werkzeughaltevorrichtung 18 ist operativ mit einer Antriebsvorrichtung 4 verbunden, so dass die Werkzeughaltevorrichtung 18 in eine Antriebsbewegung, vorzugsweise in eine Drehbewegung, versetzbar ist. Die Werkzeughaltevorrichtung 18 erstreckt sich entlang einer Längs- oder Drehachse 21. Ein Werkzeuglösevorrichtung 22 ist zum Lösen des Werkzeugs aus der Werkzeughaltevorrichtung 18 vorgesehen. Eine Werkzeugaufnahmeöffnung 23 am Kopfteil 17 ermöglicht das Einfügen oder Entnehmen des Werkzeugs in / aus der Werkzeughaltevorrichtung 18.

Die Antriebsvorrichtung 4 umfasst ein Laufrad 4A, das mit einem Antriebsfluid, insbesondere mit einem Druckgas, in Drehung versetzbar ist. Eine Fluidleitung oder Treibgasleitung 24 in dem Handstück 1 ist ausgebildet, dem Laufrad 4A das Antriebsfluid zuzuleiten. Die Antriebsvorrichtung 4, insbesondere das Laufrad 4A, und die Werkzeughaltevorrichtung 18 sind durch Wälzlager 25 drehbar in dem Kopfteil 17 gelagert.

Eine Kühlmittelabgabevorrichtung 26, zum Beispiel in Form von Düsen, am Kopfteil 17 ist zur Abgabe von Kühlspray auf eine Behandlungsstelle ausgebildet. Die Kühlmittelabgabevorrichtung 26 ist vorzugsweis um die Werkzeugaufnahmeöffnung 23 angeordnet.

Eine Außenhülse 2 grenzt das Handstück 1 zur Umgebung ab. Die Außenhülse 2 erstreckt sich, insbesondere im Bereich des Griffteils 19, länglich entlang einer Mittelachse 3. Die Mittelachse 3 der Außenhülse 2 ist insbesondere gewinkelt zur Längs- oder Drehachse 21 der Werkzeughaltevorrichtung 18 angeordnet.

In der Außenhülse 2 ist eine Öffnung 7 zur Aufnahme einer Beleuchtungsvorrichtung 5 vorgesehen. Die Öffnung 7 ist insbesondere an jener Seite des Handstücks 1 angeordnet, an der sich die Werkzeugaufnahmeöffnung 23 befindet. Vorzugsweise ist die Öffnung 7 in dem Kopfteil 17, insbesondere in dem Verbindungsteil 17A, vorgesehen.

Die Beleuchtungsvorrichtung 5 umfasst zumindest ein optisches Halbleiterelement 6, das auf einem keramischen Substrat oder Trägerelement 27 befestigt ist. Vorzugsweise umgibt ein, insbesondere kuppel- oder halbkugelig geformtes, Hüllelement 15 das optische Halbleiterelement 6. Das Hüllelement 15 ist bevorzugt aus Kunststoff, insbesondere aus verformbarem Kunststoff, hergestellt, und umfasst zum Beispiel eine Silikonverbindung. Eine in der Öffnung 7 der Außenhülse 2 angeordnete Kappe 14, die zum Beispiel halbkugelig oder kuppelförmig geformt ist, umgibt und schützt insbesondere das optische Halbleiterelement 6 und / oder das Hüllelement 15. Die Kappe 14 ist insbesondere durch eine transparente Kunststoffschale oder Kunststoffhartschale gebildet. An der Öffnung 7 und / oder an der Beleuchtungsvorrichtung 5, insbesondere an der Kappe 14, ist zumindest ein Dichtelement, zum Beispiel ein O-Ring, vorgesehen. Ein weiteres Dichtmittel oder Dichtelement 16 ist vorzugsweise an der Kappe 14 und / oder an dem Hüllelement 15 und / oder zwischen der Kappe 14 und dem Hüllelement 15 vorgesehen. Das Dichtmittel oder Dichtelement 16 umfasst zum Beispiel eine Silikonmasse oder eine silikonhältige Klebemasse.

Die Beleuchtungsvorrichtung 5 ist vorzugsweise auf einer gedruckten Leiterbahn 13 und / oder auf einem flexiblen oder biegsamen Trägerelement befestigt, insbesondere auf einer flexiblen oder biegsamen Platine. Insbesondere verbindet die gedruckte Leiterbahn 13 die Beleuchtungsvorrichtung 5 elektrisch mit einer elektrischen Energiequelle. Vorzugsweise erstreckt sich die gedruckte Leiterbahn 13 zumindest durch einen Abschnitt des Handstücks 1. Vorzugsweise umfasst die gedruckte Leiterbahn 13 zwei gewinkelt zueinander angeordnete Abschnitte, wobei auf einem der beiden Abschnitte die Beleuchtungsvorrichtung 5 befestigt ist.

Zur Befestigung und / oder Positionierung der Beleuchtungsvorrichtung 5 in dem Handstück 1, insbesondere an oder in der Öffnung 7, ist ein Stift 8 vorgesehen, der gewinkelt zur Mittelachse 3 des Handstücks 1 angeordnet ist. Der Stift 8 umfasst insbesondere Kunststoff oder ist als Kunststoffstift ausgebildet. Der Stift 8 drückt erfindungsgemäss die Beleuchtungsvorrichtung 5 in die Öffnung 7. Insbesondere ist der Stift 8 zwischen der Außenhülse 2 und der Beleuchtungsvorrichtung 5 geklemmt. Besonders bevorzugt kontaktiert der Stift 8 die gedruckte Leiterplatte 13, auf welcher die Beleuchtungsvorrichtung 5 vorgesehen ist. Insbesondere drückt der Stift 8 das Hüllelement 15 der Beleuchtungsvorrichtung 5 unter Verformung in die Kappe 14.

Der Stift 8 ist insbesondere in einer Aufnahme 9 angeordnet oder aufgenommen, die ebenfalls gewinkelt zur Mittelachse 3 des Handstücks 1 angeordnet ist. Die Aufnahme 9 umfasst vorzugsweise mehrere voneinander getrennte Abschnitte 9A, 9B, so dass insbesondere nur Teile des Stifts 8 in der Aufnahme 9 aufgenommen oder gelagert sind. Vorzugsweise sind Teile des Stifts 8 (direkt) in einem Innenraum 10 der Außenhülse 2 aufgenommen. Besonders bevorzugt sind unterschiedliche Abschnitte des Stifts 8 in zumindest einem Abschnitt der Aufnahme 9 und (direkt) in einem Innenraum 10 der Außenhülse 2 aufgenommen. Vorzugsweise ist die Außenhülse 2 als Fluidleitung 11 ausgebildet, insbesondere als Leitung, die ausgebildet ist, das Treibgas, welches die Antriebsvorrichtung 4 passiert hat, von der Antriebsvorrichtung 4 abzuleiten, so dass der Stift 8 insbesondere in einer Fluidleitung 11 oder Rückleitung des Antriebsgases angeordnet ist.

Ein Abschnitt oder eine Querbohrung 9B der Aufnahme 9 ist in einem Fortsatz 12 vorgesehen, der in den durch die Außenhülse 2 gebildeten Innenraum 10 oder in einen Innenraum der den Stift 8 aufnehmenden Fluidleitung 11 ragt. Insbesondere ist der Fortsatz 12 integral mit der Außenhülse 2 und / oder mit dem in den Griffteil 19 einfügbaren Verbindungsteil 17A verbunden und / oder der Fortsatz 12 entspringt einer Innenwand der Außenhülse 2 und / oder des Verbindungsteils 17A. Wie insbesondere aus der Figur 2 ersichtlich ist, ist der Innenraum 10 der Außenhülse 2 und / oder der Fluidleitung 11 im Querschnitt an der Seite des Fortsatzes 12 vorzugsweise in etwa S-förmig gebogen. Vorzugsweise ist in dem Fortsatz 12 zumindest teilweise eine Fluidleitung 24, zum Beispiel zur Leitung des Antriebsgases zur Antriebsvorrichtung 4, angeordnet. Vorzugsweise ist in einer Umfangswand 20 des Verbindungsteils 17A, an welcher der Fortsatz 12 vorgesehen ist, zumindest eine Fluidleitung 28 angeordnet, zum Beispiel eine Leitung zur Förderung eines Kühlmittels.

Ein weiterer Abschnitt oder eine weitere Querbohrung 9A der Aufnahme 9 ist in der Umfangswand 20 des Verbindungsabschnitts 17A, insbesondere jenes Teils des Verbindungsabschnitts 17A, der in den Griffteil 19 einfügbar ist, vorgesehen. Dieser Abschnitt 9A endet vorzugsweise in einer Öffnung 29 an der Außenseite der Umfangswand 20, so dass der Abschnitt 9A der Aufnahme 9 insbesondere als Durchbohrung der Umfangswand 20 ausgebildet ist. Damit ist es möglich, dass, wenn der Stift 8 in der Aufnahme 9 oder der Querbohrung 9A aufgenommen ist, (durch das und / oder nach dem Verbinden des Kopfteils 17 mit dem Griffteil 19) der Stift 8 durch die Außenhülse 2 in Richtung der Beleuchtungsvorrichtung 5 gedrückt wird und / oder der Stift 8 zwischen der Außenhülse 2 und der Beleuchtungsvorrichtung 5 klemmbar ist oder geklemmt wird. Vorzugsweise ragt zumindest ein Teil des Stifts 8 in die Öffnung 29 und / oder darüber hinaus.

Vorzugsweise sind die Beleuchtungsvorrichtung 5 und / oder der Stift 8 und / oder die Aufnahme 9 im Wesentlichen in dem Kopfteil 17, insbesondere in dem Verbindungsteil 17A, vorgesehen.

## Patentansprüche

1. Medizinisches, insbesondere dentales, Handstück (1), umfassend: Eine Außenhülse (2) mit einer Mittelachse (3), eine Antriebsvorrichtung (4) zum in Bewegung versetzen eines mit dem Handstück (1) verbindbaren Werkzeugs und eine Beleuchtungsvorrichtung (5) zum Beleuchten des Werkzeugs und / oder einer Behandlungsstelle, auf welche das Werkzeug einwirkt, wobei die Beleuchtungsvorrichtung (5) ein optisches Halbleiterelement (6) sowie elektrische Kontakte zur Verbindung des optischen Halbleiterelements (6) mit einer Energiequelle aufweist und an einer Öffnung (7) der Außenhülse (2) angeordnet ist, **gekennzeichnet durch**
einen Stift (8), der zumindest das optische Halbleiterelement (6) oder die Beleuchtungsvorrichtung (5) an oder in der Öffnung (7) der Außenhülse (2) fixiert, wobei der Stift (8) zumindest teilweise in einer gewinkelt zur Mittelachse (3) angeordneten Aufnahme (9, 9A, 9B) des Handstücks (1) angeordnet ist und wobei der Stift (8) die Beleuchtungsvorrichtung (5) in die Öffnung (7) drückt.

2. Medizinisches, insbesondere dentales, Handstück (1) nach Anspruch 1, **dadurch gekennzeichnet, dass**
der Stift (8) zumindest teilweise in einem durch die Außenhülse (2) gebildeten Innenraum (10) des Handstücks (1) angeordnet ist.

3. Medizinisches, insbesondere dentales, Handstück (1) nach Anspruch 2, **dadurch gekennzeichnet, dass**
der Stift (8) im Wesentlichen den gesamten durch die Außenhülse (2) gebildeten Innenraum (10) durchquert.

4. Medizinisches, insbesondere dentales, Handstück (1) nach Anspruch 1 oder 2, **dadurch gekennzeichnet, dass**
der Stift (8) zumindest teilweise in einer Fluidleitung (11) des Handstücks (1) aufgenommen ist.

5. Medizinisches, insbesondere dentales, Handstück (1) nach einem der Ansprüche 2 - 4, **gekennzeichnet durch**
einen Fortsatz (12), in dem zumindest ein Abschnitt der Aufnahme (9, 9A, 9B) für den Stift (8) vorgesehen ist, wobei der Fortsatz (12) in den durch die Außenhülse (2) gebildeten Innenraum (10) oder in einen Innenraum der den Stift (8) aufnehmenden Fluidleitung (11) ragt.

6. Medizinisches, insbesondere dentales, Handstück (1) nach einem der vorstehenden Ansprüche, **dadurch gekennzeichnet, dass**
der Stift (8) zwischen der Außenhülse (2) und der Beleuchtungsvorrichtung (5) geklemmt ist.

7. Medizinisches, insbesondere dentales, Handstück (1) nach einem der vorstehenden Ansprüche, **dadurch gekennzeichnet, dass**
der Stift (8) eine gedruckte Leiterplatte (13) kontaktiert, auf welcher die Beleuchtungsvorrichtung (5) vorgesehen ist.

8. Medizinisches, insbesondere dentales, Handstück (1) nach Anspruch 7, **dadurch gekennzeichnet, dass**
die Beleuchtungsvorrichtung (5) und der Stift (8) an gegenüberliegenden Seiten der gedruckten Leiterplatte (13) vorgesehen sind.

9. Medizinisches, insbesondere dentales, Handstück (1) nach einem der vorstehenden Ansprüche, **dadurch gekennzeichnet, dass**
der Stift (8) Kunststoff aufweist.

10. Medizinisches, insbesondere dentales, Handstück (1) nach einem der vorstehenden Ansprüche, **dadurch gekennzeichnet, dass**
die Beleuchtungsvorrichtung (5) eine in der Öffnung (7) der Außenhülse (2) angeordnete Kappe (14) aufweist.

11. Medizinisches, insbesondere dentales, Handstück (1) nach Anspruch 10, **dadurch gekennzeichnet, dass**
die Beleuchtungsvorrichtung (5) ein das optische Halbleiterelement (6) umgebendes Hüllelement (15), insbesondere ein Silikonelement, aufweist, welches durch den Stift (8) unter Verformung in die Kappe (14) gepresst ist.

12. Medizinisches, insbesondere dentales, Handstück (1) nach Anspruch 10 oder 11, **dadurch gekennzeichnet, dass**
an der Kappe (14) und / oder an dem das optische Halbleiterelement (6) umgebenden Hüllelement (15) und / oder zwischen der Kappe (14) und dem Hüllelement (15) ein Dichtmittel oder Dichtelement (16) vorgesehen ist.

13. Medizinisches, insbesondere dentales, Handstück (1) nach einem der vorstehenden Ansprüche, **dadurch gekennzeichnet, dass**
das Handstück (1) einen Kopfteil (17) mit einer Werkzeughaltevorrichtung (18) zum Halten des mit dem Handstück (1) verbindbaren Werkzeugs und einen an den Kopfteil (17) anschließenden Griffteil (19) aufweist, wobei die Beleuchtungsvorrichtung (5) und der Stift (8) im Wesentlichen in dem Kopfteil (17) vorgesehen sind.

14. Medizinisches, insbesondere dentales, Handstück (1) nach Anspruch 13, **dadurch gekennzeichnet, dass**
das Kopfteil (17) von dem Griffteil (19) lösbar ist, wobei durch das Verbinden des Kopfteils (17) mit dem Griffteil (19) der Stift (8) zwischen der Außenhülse (2) und der Beleuchtungsvorrichtung (5) klemmbar ist.

15. Medizinisches, insbesondere dentales, Handstück (1) nach Anspruch 13 oder 14, **dadurch gekennzeichnet, dass**
das Kopfteil (17) einen Verbindungsabschnitt (17A) aufweist, der zumindest teilweise in den Griffteil (19) einfügbar ist, wobei in einer Umfangswand (20) des Verbindungsabschnitts (17A) zumindest ein Abschnitt der Aufnahme (9, 9A, 9B) für den Stift (8) vorgesehen ist.

## Claims

1. A medical, in particular dental, handpiece (1) comprising: an outer case (2) with a central axis (3), a drive device (4) for setting in motion a tool which can be connected to the handpiece (1) and an illumination device (5) to illuminate the tool and/or a treatment site on which the tool is acting, wherein the illumination device (5) comprises an optical semiconductor element (6) as well as electrical contacts to connect the optical semiconductor element (6) to an energy source and wherein the illumination device (5) is disposed at an opening (7) of the outer case (2), **characterized by**
a pin (8) which fixes at least the optical semiconductor element (6) or the illumination device (5) at or in the opening (7) of the outer case (2), wherein at least a portion of the pin (8) is disposed in a receptacle (9, 9A, 9B) of the handpiece (1) which is disposed at an angle to the central axis (3) and wherein the pin (8) pushes the illumination device (5) into the opening (7).

2. The medical, in particular dental, handpiece (1) according to claim 1, **characterized in that**
at least a portion of the pin (8) is disposed in an interior space (10) of the handpiece (1) formed by the outer case (2).

3. The medical, in particular dental, handpiece (1) according to claim 2, **characterized in that**
the pin (8) essentially passes completely through the whole of the interior space (10) formed by the outer case (2).

4. The medical, in particular dental, handpiece (1) according to claim 1 or claim 2, **characterized in that**
at least a portion of the pin (8) is accommodated in a fluid line (11) of the handpiece (1).

5. The medical, in particular dental, handpiece (1) according to one of claims 2 to 4, **characterized by**
an extension (12) in which at least a section of the receptacle (9, 9A, 9B) for the pin (8) is provided, wherein the extension (12) protrudes into the interior space (10) formed by the outer case (2) or into an interior space of the fluid line (11) accommodating the pin (8).

6. The medical, in particular dental, handpiece (1) according to one of the preceding claims, **characterized in that**
the pin (8) is clamped between the outer case (2) and the illumination device (5).

7. The medical, in particular dental, handpiece (1) according to one of the preceding claims, **characterized in that**
the pin (8) contacts a printed circuit board (13) on which the illumination device (5) is provided.

8. The medical, in particular dental, handpiece (1) according to claim 7, **characterized in that**
the illumination device (5) and the pin (8) are provided on opposite sides of the printed circuit board (13).

9. The medical, in particular dental, handpiece (1) according to one of the preceding claims, **characterized in that**
the pin (8) comprises plastic.

10. The medical, in particular dental, handpiece (1) according to one of the preceding claims, **characterized in that**
the illumination device (5) comprises a cap (14) which is disposed in the opening (7) of the outer case (2).

11. The medical, in particular dental, handpiece (1) according to claim 10, **characterized in that**
the illumination device (5) comprises a cover element (15), in particular a silicone element, which surrounds the optical semiconductor element (6) and which is pushed into the cap (14) by the pin (8), thereby being deformed.

12. The medical, in particular dental, handpiece (1) according to claim 10 or claim 11, **characterized in that**
a sealing means or sealing element (16) is provided at the cap (14) and/or at the cover element (15) surrounding the optical semiconductor element (6) and/or between the cap (14) and the cover element (15).

13. The medical, in particular dental, handpiece (1) according to one of the preceding claims, **characterized in that**
the handpiece (1) comprises a headpiece (17) with a tool retaining device (18) for retaining the tool which can be connected to the handpiece (1) and a handle part (19) connected to the headpiece (17), wherein the illumination device (5) and the pin (8) are essentially provided in the headpiece (17).

14. The medical, in particular dental, handpiece (1) according to claim 13, **characterized in that**
the headpiece (17) is detachable from the handle part (19) wherein, by connecting the headpiece (17) to the handle part (19), the pin (8) can be clamped between the outer case (2) and the illumination device (5).

15. The medical, in particular dental, handpiece (1) as claimed claim 13 or claim 14, **characterized in that**
the headpiece (17) comprises a connecting section (17A), at least a portion of which can be inserted into the handle part (19), wherein at least a section of the receptacle (9, 9A, 9B) for the pin (8) is provided in a circumferential wall (20) of the connecting section (17A).

## Revendications

1. Pièce à main (1) médicale, en particulier dentaire, comprenant: un manchon extérieur (2) avec un axe central (3), un dispositif d'entraînement (4) pour mettre en mouvement un outil pouvant être relié à la pièce à main (1) et un dispositif d'éclairage (5) pour éclairer l'outil et/ou un site de traitement, sur lequel l'outil agit, dans laquelle le dispositif d'éclairage (5) présente un élément semi-conducteur optique (6) ainsi que des contacts électriques pour relier l'élément semi-conducteur optique (6) à une source d'énergie, et est disposé à une ouverture (7) du manchon extérieur (2), **caractérisée par**
une broche (8) qui fixe au moins l'élément semi-conducteur optique (6) ou le dispositif d'éclairage (5) à l'ouverture (7), ou dans celle-ci, du manchon extérieur (2), dans laquelle la broche (8) est disposée au moins partiellement dans un logement (9, 9A, 9B), disposé en angle par rapport à l'axe central (3), de la pièce à main (1) et dans laquelle la broche (8) pousse le dispositif d'éclairage (5) dans l'ouverture (7).

2. Pièce à main (1) médicale, en particulier dentaire selon la revendication 1, **caractérisée en ce que**
la broche (8) est disposée au moins partiellement dans un espace intérieur (10) de la pièce à main (1) formé par le manchon extérieur (2).

3. Pièce à main (1) médicale, en particulier dentaire selon la revendication 2, **caractérisée en ce que**
la broche (8) traverse essentiellement l'ensemble de l'espace intérieur (10) formé par le manchon extérieur (2).

4. Pièce à main (1) médicale, en particulier dentaire selon la revendication 1 ou 2, **caractérisée en ce que**
la broche (8) est au moins partiellement réceptionnée dans une conduite de fluide (11) de la pièce à main (1).

5. Pièce à main (1) médicale, en particulier dentaire selon l'une des revendications 2 - 4, **caractérisée par**
un prolongement (12) dans lequel est prévue au moins une partie du logement (9, 9A, 9B) pour la broche (8), dans laquelle le prolongement (12) fait saillie dans l'espace intérieur (10) formé par le manchon extérieur (2) ou dans un espace intérieur de la conduite de fluide (11) réceptionnant la broche (8).

6. Pièce à main (1) médicale, en particulier dentaire selon l'une des revendications précédentes, **caractérisée en ce que**
la broche (8) est serrée entre le manchon extérieur (2) et le dispositif d'éclairage (5).

7. Pièce à main (1) médicale, en particulier dentaire selon l'une des revendications précédentes, **caractérisée en ce que**
la broche (8) entre en contact avec une carte de circuit imprimée (13) sur laquelle le dispositif d'éclairage (5) est prévu.

8. Pièce à main (1) médicale, en particulier dentaire selon la revendication 7, **caractérisée en ce que**
le dispositif d'éclairage (5) et la broche (8) sont prévus sur des côtés situés en vis-à-vis de la carte de circuit imprimé (13).

9. Pièce à main (1) médicale, en particulier dentaire selon l'une des revendications précédentes, **caractérisée en ce que**
la broche (8) présente du plastique.

10. Pièce à main (1) médicale, en particulier dentaire selon l'une des revendications précédentes, **caractérisée en ce que**
le dispositif d'éclairage (5) présente un capuchon (14) disposé dans l'ouverture (7) du manchon extérieur (2).

11. Pièce à main (1) médicale, en particulier dentaire selon la revendication 10, **caractérisée en ce que**
le dispositif d'éclairage (5) présente un élément enveloppant (15) entourant l'élément semi-conducteur optique (6), en particulier un élément en silicone, lequel est pressé en se déformant dans le capuchon (14) par la broche (8).

12. Pièce à main (1) médicale, en particulier dentaire selon la revendication 10 ou 11, **caractérisée en ce que**
l'on prévoit, sur le capuchon (14) et/ou l'élément enveloppant (15) entourant l'élément semi-conducteur optique (6) et/ou entre le capuchon (14) et l'élément enveloppant (15), un moyen d'étanchéification ou un élément d'étanchéification (16).

13. Pièce à main (1) médicale, en particulier dentaire selon l'une des revendications précédentes, **caractérisée en ce que**
la pièce à main (1) présente une partie de tête (17) avec un dispositif porte-outil (18) pour porter l'outil pouvant être relié à la pièce à main (1) et une partie de manche (19) en jonction avec la partie de tête (17), dans laquelle le dispositif d'éclairage (5) et la broche (8) sont essentiellement prévus dans la partie de tête (17).

14. Pièce à main (1) médicale, en particulier dentaire selon la revendication 13, **caractérisée en ce que**
la partie de tête (17) est détachable de la partie de manche (19), dans laquelle, en reliant la partie de tête (17) à la partie de manche (19), la broche (8) peut être serrée entre le manchon extérieur (2) et le dispositif d'éclairage (5).

15. Pièce à main (1) médicale, en particulier dentaire selon la revendication 13 ou 14, **caractérisée en ce que**
la partie de tête (17) présente une partie de liaison (17A) pouvant être au moins partiellement insérée dans la partie de manche (19), dans laquelle on prévoit, dans une paroi périphérique (20) de la partie de liaison (17A), au moins une partie du logement (9, 9A, 9B) pour la broche (8).
